# FASCICULE DE BREVET EUROPEEN

(11) **EP 2 694 501 B1**
(45) Date de publication et mention de la délivrance du brevet: **17.12.2014**
(21) Numéro de dépôt: 12711161.5
(22) Date de dépôt: 28.03.2012
(51) Int. Cl.: C07D 471/04, C22B 60/02, C22B 3/00, G21C 19/42

(54) **DÉRIVÉS DE LA 2,9-DIPYRIDYL-1,10-PHENANTHROLINE UTILES COMME LIGANDS DES ACTINIDES, LEUR PROCÉDÉ DE SYNTHÈSE ET LEURS UTILISATIONS**
2,9-DIPYRIDYL-1,10-PHENANTHROLINDERIVATE ALS ACTINIDLIGANDEN, VERFAHREN ZU IHRER HERSTELLUNG UND IHRE VERWENDUNG
2,9-DIPYRIDYL-1,10-PHENANTHROLINE DERIVATIVES USEFUL AS ACTINIDE LIGANDS, METHOD FOR SYNTHESIZING SAME, AND USES THEREOF

(30) Priorité: 01.04.2011 FR 1152835
(43) Date de publication de la demande: 12.02.2014
(73) Titulaire: Commissariat à l'Énergie Atomique et aux Énergies Alternatives, 75015 Paris (FR); Université de Nantes, 44035 Nantes Cedex 1 (FR)
(72) Inventeur: BISSON, Julia, F-50100 CHERBOURG (FR); CHARBONNEL, Marie-Christine, F-30200 Saint Gervais (FR); BOUBALS, Nathalie, F-84857 Camaret D'aigues (FR); MIGUIRDITCHIAN, Manuel, F-84000 Avignon (FR); GUILLANEUX, Denis, F-30400 Villeneuve D'avignon (FR); GUILLAUMONT, Dominique, F-84000 Avignon (FR); MARIE, Cécile, F-28170 Chateauneuf en Thymerais (FR); DUBREUIL, Didier, F-44710 Port Saint Père (FR); PIPELIER, Muriel, F-44300 Nantes (FR); BLOT, Virginie, F-44700 Orvault (FR)
(74) Mandataire: Ahner, Philippe
(86) Numéro de dépôt international: PCT/EP2012/055557
(87) Numéro de publication internationale: WO 2012/130902

(56) Documents cités:
- WO-A1-2011/009814
- WO-A1-2011/030566
- WO-A2-2007/022307

## Description

### DOMAINE TECHNIQUE

La présente invention se rapporte à de nouveaux composés, qui sont des dérivés de la 2,9-dipyridyl-1,10-phénanthroline et qui sont utiles comme ligands des actinides.

Elle se rapporte également à un procédé permettant de synthétiser ces composés ainsi qu'à leurs utilisations.

Les composés selon l'invention sont capables d'extraire seuls, c'est-à-dire en l'absence de toute autre molécule extractante, les actinides(III), (IV), (V) et (VI) d'une solution aqueuse fortement acide beaucoup plus efficacement qu'ils n'extraient les lanthanides.

Ils sont donc, à ce titre, susceptibles d'être utilisés pour séparer de façon groupée l'ensemble des actinides (uranium, neptunium, plutonium, américium et/ou curium) présents dans une solution aqueuse acide, telle qu'une solution de dissolution d'un combustible nucléaire usé, des lanthanides se trouvant également dans cette solution.

De plus, les dérivés selon l'invention présentent une affinité plus marquée pour l'américium que pour le curium.

Ils sont donc également susceptibles d'être utilisés pour séparer l'américium présent dans une solution aqueuse fortement acide, telle qu'un raffinat du type de ceux produits au cours du premier cycle du procédé PUREX ou du procédé COEX™, du curium se trouvant également dans cette solution.

### ÉTAT DE LA TECHNIQUE ANTÉRIEURE

De nombreuses molécules extractantes ont été étudiées dans l'objectif de séparer les actinides présents dans des solutions de dissolution de combustibles nucléaires usés des lanthanides qui y sont également présents.

Une des grandes difficultés est de trouver des molécules qui soient capables d'extraire les actinides beaucoup plus efficacement qu'elles n'extraient les lanthanides, sachant que, d'une part, les lanthanides sont présents dans les solutions de dissolution des combustibles nucléaires usés en quantités très supérieures à celles des actinides et, d'autre part, ce type de solution présente typiquement une forte acidité, c'est-à-dire généralement égale ou supérieure à 3 mol/L d'acide nitrique.

Pour séparer de façon groupée le plutonium, le neptunium, l'américium, le curium (et éventuellement l'uranium) des lanthanides présents dans une solution de dissolution d'un combustible nucléaire usé, il a été proposé deux procédés qui utilisent plusieurs étapes dont la première est une extraction par des extractants tels qu'un mélange de malonamide comme le *N,N'-*diméthyl-*N,N*'-dioctylhexyléthoxymalonamide (ou DMDOHEMA) avec un acide alkylphosphorique comme l'acide di-(2-éthylhexyl)phosphorique (ou HDEHP), ou un diglycolamide comme le *N,N,N',N'*-tétraoctyl-3-oxa-pentanediamide (ou TODGA).

Ces procédés sont respectivement décrits dans les demandes internationales PCT publiées sous les numéros WO 2007/118904 (référence **[1]**) et WO 2008/ 049807 (référence **[2]**).

Il se trouve que les extractants qui présentent des atomes d'oxygène donneurs, tels que les malonamides et les diglycolamides, ne permettent pas d'extraire, d'une solution aqueuse acide contenant à la fois des actinides et des lanthanides, les actinides sans extraire en même temps les lanthanides.

De ce fait, les procédés décrits dans les références précitées comprennent tout d'abord une étape visant à co-extraire les actinides et les lanthanides de la solution aqueuse dans laquelle ils se trouvent, au moyen d'une phase organique qui contient le malonamide ou le diglycolamide.

Cette étape de co-extraction est suivie d'une étape visant à désextraire sélectivement les actinides de la phase organique, que l'on réalise au moyen d'une phase aqueuse faiblement acide, c'est-à-dire d'un pH compris entre 2 et 3, et contenant un agent complexant, par exemple un acide polyamino-carboxylique. Les lanthanides sont alors retenus dans la phase organique soit grâce à la présence, dans cette phase organique, d'un extractant acide du type acide phosphorique (référence **[1]**) soit grâce à la présence, dans la phase aqueuse faiblement acide, d'ions nitrate (référence **[2]**).

Vient ensuite une étape visant à désextraire les lanthanides de la phase organique pour, d'une part, récupérer ces lanthanides dans une phase aqueuse propre à être soumise ultérieurement aux opérations de vitrification, et, d'autre part, épurer la phase organique en radioéléments en vue de sa réutilisation.

Par ailleurs, on connaît des composés qui présentent une affinité plus grande pour les actinides, et en particulier pour les actinides(III), que pour les lanthanides.

Il s'agit de composés polyaromatiques azotés comme la 2,2':6',2"-terpyridine et certains de ses dérivés alkylés, la 2,4,6-tri(2-pyridinyl)-1,3,5-triazine (ou TPTZ), la 2,6-bis(pyridin-2-yl)-4-amino-1,3,5-triazine (ou ADPTZ) et les 2,6-bis(1,2,4-triazinyl)pyridines, de picolinamides, de dipicolinamides et de bipyridines à substitutions amide.

Toutefois, aucun de ces composés n'apparaît susceptible d'être utilisé dans un procédé industriel qui viserait à séparer de façon groupée les actinides présents dans des solutions de dissolution de combustibles nucléaires usés des lanthanides se trouvant également dans ces solutions.

En effet :
- soit ces composés sont tout simplement incapables d'extraire seuls (c'est-à-dire en l'absence d'une autre molécule extractante) les actinides d'une phase aqueuse, et notamment d'une phase aqueuse fortement acide, ce qui est le cas, par exemple, de la 2,2': 6', 2"-terpyridine et de ses dérivés alkylés, de la TPTZ, de l'ADPTZ et des picolinamides qui n'extraient les actinides qu'à faible acidité et en mélange synergique avec un autre extractant, typiquement, l'acide α-bromodécanoïque ;
- soit ces composés présentent une capacité de charge trop faible, ce qui est le cas, par exemple, des 2,6-bis(1,2,4-triazinyl)pyridines ;
- soit ils nécessitent d'être en solution dans un diluant polaire, halogéné et toxique, comme le chloroforme ou le *méta*-nitrotrifluorotoluène, ce qui est le cas, par exemple, des dipicolinamides ; or, ce type de diluant n'est pas utilisable dans un procédé industriel de traitement des combustibles nucléaires usés ;
- soit encore ils sont proposés avec ajout d'un extractant donneur oxygéné tel que le TBP (référence **[3],** mais l'inconvénient est la gestion d'une solution organique multicomposant ainsi que la sélection d'un diluant tel que la cyclohexanone, peu compatible avec une exploitation à une échelle industrielle.

Très récemment, il a été montré, dans la demande internationale PCT publiée sous le numéro WO 2011/009814 (référence **[4]**), que des terpyridines porteuses de groupes amides sont susceptibles d'être utilisées, en solution dans du *n*-octanol, pour séparer l'ensemble des actinides (III), (IV), (V) et (VI) présents dans une solution aqueuse fortement acide ([HNO₃] = 3 mol/L) des lanthanides se trouvant également dans cette solution.

Toutefois, les coefficients de distribution des actinides(III), c'est-à-dire de l'américium et du curium, obtenus avec ces terpyridines sont relativement faibles (≤ à 0,018), ce qui signifie que, si ces molécules devaient être utilisées pour séparer l'ensemble des actinides des lanthanides dans le cadre d'un procédé industriel de traitement des combustibles nucléaires usés, il conviendrait d'employer un nombre d'étages d'extraction assez élevé pour garantir une séparation totale des actinides(III) des lanthanides.

Les Inventeurs se sont donc fixé pour but de trouver de nouveaux composés qui, non seulement soient capables d'extraire l'ensemble des actinides présents dans une solution aqueuse acide, et notamment dans une solution aqueuse d'acidité élevée, plus efficacement qu'ils n'extraient les lanthanides, mais permettent de plus d'obtenir, pour les actinides(III), des coefficients de distribution significativement plus élevés que ceux obtenus pour ces mêmes actinides dans la référence **[4]** précitée.

Les Inventeurs se sont aussi fixé pour but que ces composés puissent être employés en solution dans un diluant non aqueux compatible avec un procédé industriel de traitement de combustibles nucléaires usés.

### EXPOSÉ DE L'INVENTION

Ces buts et d'autres encore sont atteints par l'invention qui propose, en premier lieu, un composé qui répond à la formule générale (I) ci-après : 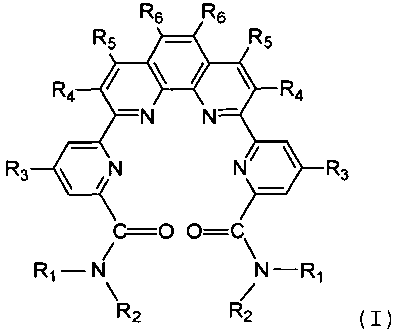 dans laquelle :
R₁ et R₂, identiques ou différents, représentent un atome d'hydrogène, un groupe hydrocarboné linéaire ou ramifié, saturé ou insaturé, en C₁ à C₁₂, ou un groupe aryle ou aryl-alkyle en C₁ à C₆ monocyclique ;
R₃, R₄, R₅ et R₆, identiques ou différents, représentent :
   * un atome d'hydrogène ; ou
   * un groupe hydrocarboné linéaire ou ramifié, saturé ou insaturé, en C₁ à C₁₂ ; ou
   * un groupe aryle ou aryl-alkyle en C₁ à C₆ monocyclique ; ou
   * un groupe -NR₇R₈ ou -NR₇COR₈ dans lequel R₇ représente un atome d'hydrogène, un groupe hydrocarboné linéaire ou ramifié, saturé ou insaturé, en C₁ à C₁₂, ou un groupe aryle ou aryl-alkyle en C₁ à C₆ monocyclique, tandis que R₈ représente un groupe hydrocarboné linéaire ou ramifié, saturé ou insaturé, en C₁ à C₁₂, ou un groupe aryle ou aryl-alkyle en C₁ à C₆ monocyclique ; ou encore
   * un groupe -OR₉ ou -SR₉ dans lequel R₉ représente un groupe hydrocarboné linéaire ou ramifié, saturé ou insaturé, en C₁ à C₁₂, ou un groupe aryle ou aryl-alkyle en C₁ à C₆ monocyclique.

Ainsi, le composé selon l'invention est un ligand bifonctionnel hexadenté, qui comprend à la fois un motif polyhétérocyclique azoté, en l'espèce un motif 2,9-dipyridyl-1,10-phénanthroline, et deux groupes amides -CONR₁R₂, respectivement portés par les groupes pyridyle dudit motif polyhétérocyclique azoté.

Dans ce qui précède et ce qui suit, on entend par « groupe hydrocarboné saturé ou insaturé, linéaire ou ramifié, en C₁ à C₁₂ », tout groupe alkyle, à chaîne linéaire ou ramifiée, qui comprend au moins 1 atome de carbone mais pas plus de 12 atomes de carbone et tout groupe alcényle ou alcynyle, à chaîne linéaire ou ramifiée, qui comprend au moins 2 atomes de carbone et pas plus de 12 atomes de carbone.

De tels groupes hydrocarbonés sont, par exemple, les groupes méthyle, éthyle, *n*-propyle, isopropyle, butyle comme *n*-butyle, *sec*-butyle, isobutyle ou *tert*-butyle, pentyle comme *n*-pentyle, *sec-*pentyle ou isopentyle, hexyle comme *n*-hexyle ou isohexyle, octyle comme *n*-octyle ou isooctyle, décyle comme *n*-décyle ou isodécyle, dodécyle, éthylényle, propylényle, butényle, pentényle, hexényle, méthyl-pentényle, buta-1,3-diényle, octényle, décényle, dodécényle, éthynyle, propynyle, butynyle, pentynyle, hexynyle, octynyle, décynyle, dodécynyle, etc.

Par ailleurs, on entend par « groupe aryle monocyclique », tout groupe qui ne comprend qu'un seul cycle et dont le cycle répond à la règle d'aromaticité de Hückel et présente donc un nombre d'électrons π délocalisés égal à 4*n* + 2, tandis qu'on entend par « groupe aryl-alkyle en C₁ à C₆ monocyclique », tout groupe alkyle, linéaire ou ramifié, qui comprend au moins un atome de carbone mais pas plus de 6 atomes de carbone et dont l'un des atomes de carbone est substitué par un groupe aryle tel que précédemment défini.

De tels groupes aryle sont, par exemple, le groupe phényle et les groupes *ortho-, méta-* et *para-*tolyle tandis que de tels groupes arylalkyle sont, par exemple, les groupes benzyle, phénéthyle, phényl-propyle, phénylbutyle, phénylpentyle, phénylhexyle, *ortho-, méta-* et *para*-tolylméthyle, *ortho-, méta-* et *para*-tolyléthyle, *ortho-, méta*- et *para*-tolylpropyle, *ortho-, méta-* et *para*-tolylbutyle, *ortho-, méta*- et *para*-tolylpentyle, et *ortho-, méta-* et *para*-tolyl-hexyle.

Conformément à l'invention, le composé répond, de préférence, à la formule générale (I) ci-avant dans laquelle :
R₁ et R₂, identiques ou différents, représentent un atome d'hydrogène, un groupe alkyle linéaire ou ramifié en C₁ à C₁₂ ou un groupe aryle monocyclique ;
R₃, R₄, R₅ et R₆, identiques ou différents, représentent :
   * un atome d'hydrogène ; ou
   * un groupe alkyle linéaire ou ramifié en C₁ à C₁₂ ; ou
   * un groupe aryle monocyclique ; ou
   * un groupe -NR₇R₈ ou -NR₇COR₈ dans lequel R₇ représente un atome d'hydrogène, un groupe alkyle linéaire ou ramifié en C₁ à C₁₂, ou un groupe aryle monocyclique, tandis que R₈ représente un groupe alkyle linéaire ou ramifié en C₁ à C₁₂ ou un groupe aryle monocyclique ; ou encore
   * un groupe -OR₉ ou -SR₉ dans lequel R₉ représente un groupe alkyle linéaire ou ramifié en C₁ à C₁₂, ou un groupe aryle monocyclique.

Parmi ces composés, on préfère tout particulièrement ceux qui répondent à la formule générale (I) ci-avant dans laquelle R₃, R₄, R₅ et R₆ représentent un atome d'hydrogène pour la simple raison que la synthèse de composés dont le motif 2,9-dipyridyl-1,10-phénanthroline ne comporte aucun autre substituant que les deux groupes amides -CONR₁R₂ est généralement plus simple à mettre en oeuvre que celle de composés dont le motif 2,9-dipyridyl-1,10-phénanthroline est davantage substitué.

Egalement, on préfère tout particulièrement les composés qui répondent à la formule générale (I) ci-avant dans laquelle R₁ et R₂, identiques ou différents, représentent soit une chaîne alkyle en C₁ à C₁₂, et mieux encore en C₂ à C₁₂, soit un groupe phényle, soit encore un groupe *ortho-, méta-* ou *para*-tolyle.

De tels composés sont notamment :
- le *N,N,N',N'*-tétraéthyl-6,6'-([1,10]-phénanthrolin-2,9-diyl)-pyridin-2-yldiamide, qui répond à la formule générale (I) ci-avant dans laquelle R₁ et R₂ représentent un groupe éthyle tandis que R₃, R₄, R₅ et R₆ représentent un atome d'hydrogène ;
- le *N,N,N',N'*-tétrahexyl-6,6'-([1,10]-phénanthrolin-2,9-diyl)-pyridin-2-yldiamide, qui répond à la formule générale (I) ci-avant dans laquelle R₁ et R₂ représentent un groupe *n*-hexyle tandis que R₃, R₄, R₅ et R₆ représentent un atome d'hydrogène ;

- le *N,N,N',N'*-tétraoctyl-6,6'-([1,10]-phénanthrolin-2,9-diyl)-pyridin-2-yldiamide, qui répond à la formule générale (I) ci-avant dans laquelle R₁ et R₂ représentent un groupe *n*-octyle tandis que R₃, R₄, R₅ et R₆ représentent un atome d'hydrogène ;
- le *N,N,N',N'*-tétradécyl-6,6'-([1,10]-phénanthrolin-2,9-diyl)-pyridin-2-yldiamide, qui répond à la formule générale (I) ci-avant dans laquelle R₁ et R₂ représentent un groupe *n*-décyle tandis que R₃, R₄, R₅ et R₆ représentent un atome d'hydrogène ;
- le *N,N,N',N'*-tétradodécyl-6,6'-([1,10]-phénanthrolin-2,9-diyl)-pyridin-2-yldiamide, qui répond à la formule générale (I) ci-avant dans laquelle R₁ et R₂ représentent un groupe *n*-octyle tandis que R₃, R₄, R₅ et R₆ représentent un atome d'hydrogène ;
- le *N,N'*-diéthyl-*N,N'*-diphényl-6,6'-([1,10]-phénanthrolin-2,9-diyl)-pyridin-2-yldiamide, qui répond à la formule générale (I) ci-avant dans laquelle R₁ représente un groupe éthyle, R₂ représente un groupe phényle tandis que R₃, R₄, R₅ et R₆ représentent un atome d'hydrogène ;
- le *N,N'*-dihexyl-*N,N'*-diphényl-6,6'-([1,10]-phénanthrolin-2,9-diyl)-pyridin-2-yldiamide, qui répond à la formule générale (I) ci-avant dans laquelle R₁ représente un groupe *n*-hexyle, R₂ représente un groupe phényle tandis que R₃, R₄, R₅ et R₆ représentent un atome d'hydrogène ;
- le *N,N'*-dioctyl-*N,N'*-diphényl-6,6'-([1,10]-phénanthrolin-2,9-diyl)-pyridin-2-yldiamide, qui répond à la formule générale (I) ci-avant dans laquelle R₁ représente un groupe *n*-octyle, R₂ représente un groupe phényle tandis que R₃, R₄, R₅ et R₆ représentent un atome d'hydrogène ;
- le *N,N'*-didécyl-*N,N'*-diphényl-6,6'-([1,10]-phénanthrolin-2,9-diyl)-pyridin-2-yldiamide, qui répond à la formule générale (I) ci-avant dans laquelle R₁ représente un groupe *n*-décyle, R₂ représente un groupe phényle tandis que R₃, R₄, R₅ et R₆ représentent un atome d'hydrogène ; et
- le *N,N'*-didodécyl-*N,N'*-diphényl-6,6'-([1,10]-phénanthrolin-2,9-diyl)-pyridin-2-yldiamide, qui répond à la formule générale (I) ci-avant dans laquelle R₁ représente un groupe *n*-dodécyle, R₂ représente un groupe phényle tandis que R₃, R₄, R₅ et R₆ représentent un atome d'hydrogène.

Les composés selon l'invention peuvent notamment être obtenus par un procédé qui comprend les étapes suivantes :
- la réaction d'un composé répondant à la formule générale (II) ci-après : 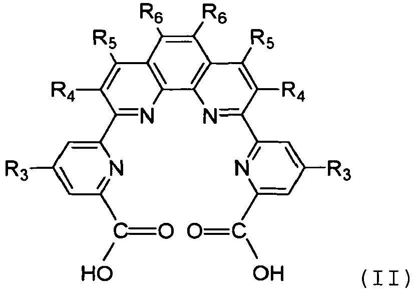 dans laquelle R₃, R₄, R₅ et R₆, identiques ou différents, ont la même signification que dans la formule générale (I) ci-avant, avec un réactif d'halogénation pour obtenir un composé répondant à la formule générale (III) ci-après : 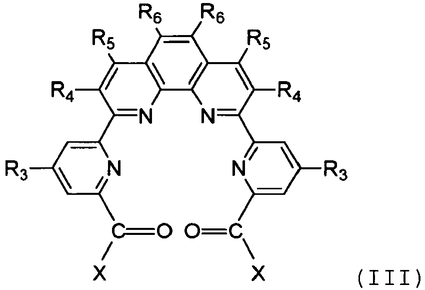 dans laquelle R₃, R₄, R₅ et R₆, identiques ou différents, ont la même signification que dans la formule générale (II) ci-avant tandis que X représente un atome de chlore; puis
- la réaction du composé répondant à la formule générale (III) ci-avant avec une amine de formule HNR₁R₂ dans laquelle R₁ et R₂ ont la même signification que dans la formule générale (I) ci-avant, cette réaction étant réalisée en présence d'une base et dans un solvant polaire aprotique.

Aussi, l'invention a-t-elle aussi pour objet un procédé de synthèse tel que décrit ci-avant.

Dans ce procédé, le réactif d'halogénation peut notamment être le chlorure de thionyle, l'oxychlorure de phosphore ou le trichlorure de phosphore ; la base peut notamment être la triéthylamine, la diisopropyléthylamine ou la pyridine tandis que le solvant polaire aprotique peut notamment être le dichlorométhane, l'acétonitrile ou le diméthylsulfoxyde.

Bien entendu, il est également possible d'obtenir les composés selon l'invention à partir d'un composé répondant à la formule générale (II) ci-avant en soumettant ce dernier à des réactions autres que celles qui viennent d'être décrites.

Ainsi, par exemple, les composés selon l'invention peuvent aussi être obtenus en faisant réagir un composé répondant à la formule générale (II) ci-avant avec une amine de formule HNR₁R₂ dans laquelle R₁ et R₂ ont la même signification que dans la formule générale (I) ci-avant, en présence d'un agent de couplage peptidique, tel que le chlorhydrate de 1-éthyl-3-[3-diméthylaminopropyl]carbodiimide, et d'un catalyseur de couplage peptidique, tel que le *N*-hydroxybenzotriazole, en solution dans un solvant organique polaire aprotique du type de ceux précités.

Dans tous les cas, le composé répondant à la formule générale (II) ci-avant peut être obtenu par :
- couplage par la méthode de Stille d'un composé de 1,10-phénanthroline substitué en positions 2,9 par un atome d'halogène, par exemple de chlore, avec un composé de 2-méthylpyridine substitué par un groupe organo-stannique, par exemple un groupe tri-*n-*butylétain, en présence d'un catalyseur palladié ; ou toute autre méthode de couplage organocatalysé pour la formation de liaisons Carbone-Carbone entre deux réactifs hétérocycliques du type couplage de Negishi, Suzuki ou Ullmann ; puis
- l'oxydation du composé ainsi obtenu au moyen d'un agent oxydant du type dioxyde de sélénium, dans un solvant polaire aprotique tel que la pyridine.

Quant au composé de 1,10-phénanthroline substitué en positions 2,9 par un atome d'halogène, il peut être obtenu à partir de la 1,10-phénanthroline par le protocole décrit par Yamada et al. dans la référence **[5].**

Les composés selon l'invention présentent une affinité particulièrement élevée pour les actinides à leurs différents degrés d'oxydation.

Plus particulièrement, les composés selon l'invention sont capables d'extraire l'ensemble des actinides(III), (IV), (V) et (VI) d'une phase aqueuse acide, et notamment d'une phase aqueuse fortement acide, comme une solution d'acide nitrique de molarité supérieure ou égale à 2.

Ces actinides peuvent notamment être l'américium(III) et le curium(III), le plutonium(IV), le neptunium(V), le neptunium(VI) et l'uranium(VI).

Aussi, l'invention a-t-elle encore pour objet l'utilisation d'un composé tel que précédemment défini en tant que ligand d'un ou plusieurs actinides et, en particulier, l'utilisation de ce composé pour extraire un ou plusieurs actinides d'une solution aqueuse acide.

Dans le cadre de ces utilisations, le composé selon l'invention peut notamment être utilisé pour séparer l'ensemble des actinides présents dans une solution aqueuse acide des lanthanides qui sont également présents dans cette solution.

Une telle solution aqueuse acide peut notamment être une solution résultant de la dissolution d'un combustible nucléaire usé dans de l'acide nitrique.

Toutefois, il peut également s'agir d'une solution résultant de la dissolution d'un combustible nucléaire usé dans de l'acide nitrique que l'on a préalablement débarrassée de l'uranium qu'elle contenait initialement.

Le composé selon l'invention peut également être utilisé pour séparer l'américium présent dans une solution aqueuse acide du curium qui est également présent dans cette solution.

Une telle solution aqueuse acide peut notamment être un raffinat du type de ceux produits au cours du premier cycle du procédé PUREX ou du procédé COEX^{™} qui contiennent de l'américium, du curium et des lanthanides mais qui ne contiennent plus ni uranium ni plutonium ni neptunium.

L'utilisation du composé selon l'invention pour séparer l'ensemble des actinides présents dans une solution aqueuse acide des lanthanides se trouvant également dans cette solution comprend typiquement les étapes suivantes :
- l'extraction de l'ensemble des actinides de la solution aqueuse acide par mise en contact de cette solution avec une phase organique comprenant ce composé puis séparation de ladite solution aqueuse et de ladite phase organique ; et
- la désextraction de l'ensemble des actinides présents dans la phase organique obtenue à l'issue de l'étape d'extraction par mise en contact de cette phase organique avec une phase aqueuse acide, de préférence faiblement acide, c'est-à-dire typiquement de pH allant de 2 à 3, et comprenant éventuellement un ou plusieurs agents complexants, puis séparation desdites phases organique et aqueuse.

Quant à l'utilisation du composé selon l'invention pour séparer l'américium présent dans une solution aqueuse acide du curium se trouvant également dans cette solution, elle comprend typiquement les étapes suivantes :
- l'extraction de l'américium de la solution aqueuse acide par mise en contact de cette solution aqueuse avec une phase organique comprenant le composé puis séparation de ladite solution aqueuse et de ladite phase organique ; et
- la désextraction de l'américium présent dans la phase organique obtenue à l'issue de l'étape d'extraction par mise en contact de cette phase organique avec une phase aqueuse acide, de préférence faiblement acide, c'est-à-dire typiquement de pH allant de 2 à 3, et comprenant éventuellement un ou plusieurs agents complexants, puis séparation desdites phases organique et aqueuse.

Dans tous les cas, le composé est avantageusement utilisé en solution, à raison de 0,001 à 0,5 mol/L, dans un diluant organique, lequel est, de préférence, choisi parmi le *n*-octanol, le *n*-dodécane et le tétrapropylène hydrogéné.

D'autres caractéristiques et avantages de l'invention apparaîtront mieux à la lecture du complément de description qui suit, qui se rapporte à des exemples de synthèse de composés selon l'invention et de démonstration de leurs propriétés extractantes.

Bien entendu, ces exemples ne sont donnés qu'à titre d'illustration de l'objet de l'invention et ne constituent en aucun cas une limitation de cet objet.

### BREVE DESCRIPTION DE LA FIGURE

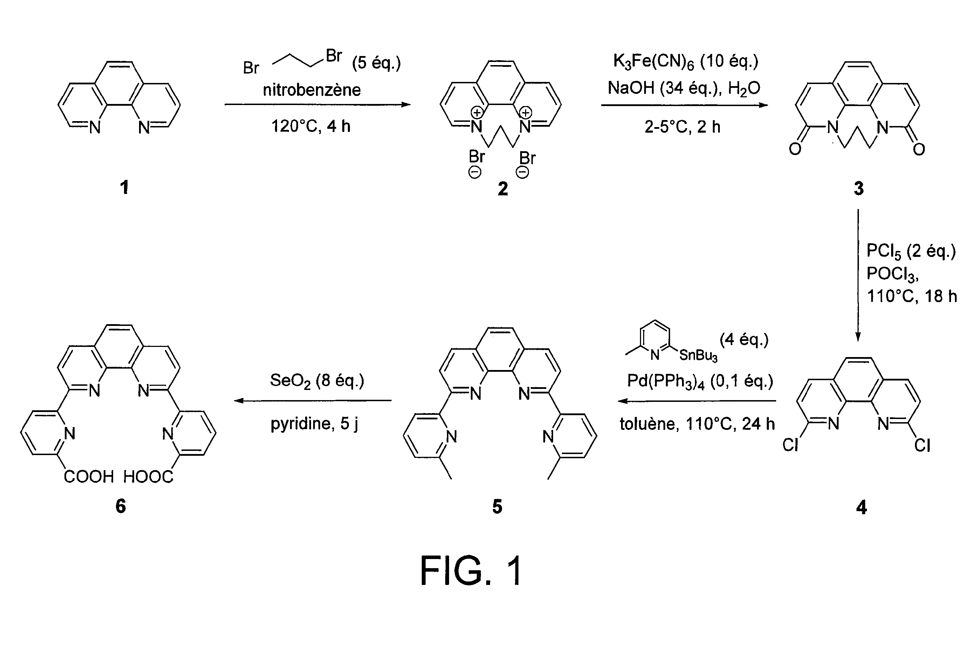

La figure 1 illustre le schéma réactionnel utilisé pour la synthèse de composés selon l'invention.

### EXPOSÉ DÉTAILLÉ DE MODES DE RÉALISATION PARTICULIERS

### EXEMPLE 1 : SYNTHESE DE COMPOSES SELON L'INVENTION

On synthétise des composés selon l'invention, à savoir :
- le *N,N,N',N'*-tétraoctyl-6,6'-([1,10]-phénanthrolin-2,9-diyl)-pyridin-2-yldiamide, noté ci-après **todi(ampyr)phen,** qui répond à la formule générale (I) ci-avant dans laquelle R₁ = R₂ = C₈H₁₇ et R₃ = R₄ = R₅ = R₆ = H ; et
- le *N,N'*-dihexyl-*N,N'*-diphényl-6,6'-([1,10]-phénanthrolin-2,9-diyl)-pyridin-2-yldiamide, noté ci-après **dihexphdi(ampyr)phen,** qui répond à la formule générale (I) ci-avant dans laquelle R₁ = C₆H₁₃, R₂ = phényle et R₃ = R₄ = R₅ = R₆ = H ;
- le *N,N,N',N'*-tétraéthyl-6,6'-([1,10]-phénanthrolin-2,9-diyl)-pyridin-2-yldiamide, noté ci-après **dietdi(ampyr)phen,** qui répond à la formule générale (I) ci-avant dans laquelle R₁ = R₂ = C₂H₅ et R₃ = R₄ = R₅ = R₆ = H ;
- le *N,N'*-diéthyl-*N,N'*-diphényl-6,6'-([1,10]-phénanthrolin-2,9-diyl)-pyridin-2-yldiamide, noté ci-après **dietphdi(ampyr)phen,** qui répond à la formule générale (I) ci-avant dans laquelle R₁ = C₂H₅, R₂ = phényle et R₃=R₄ = R₅ = R₆ = H ;
à partir de l'acide 6,6'-(2,9-dipyridyl-1,10-phénanthroline), noté ci-après composé **6.**

### 1.1. Synthèse du composé 6

Le composé **6** est préalablement obtenu à partir de la 1,10-phénanthroline, notée ci-après composé **1,** en suivant le schéma réactionnel illustré sur la figure 1.

Le composé **1** est commercialement disponible.

Les composés **2, 3** et **4** sont obtenus selon le protocole décrit par Yamada et al. dans la référence **[5]** précitée.

Ainsi, le composé **2** est obtenu en traitant le composé **1** par un large excès de 1,3-dibromopropane (5 éq.) dans le nitrobenzène à reflux, à 120°C et pendant 4 heures, puis en recristallisant le produit obtenu dans un mélange éthanol/eau 75:25. Le rendement est de 98%.

Le composé **3** est obtenu en oxydant le composé **2** par du ferricyanure de potassium (10 éq.) dans une solution aqueuse d'hydroxyde de sodium (34 éq.) à une température comprise entre 2 et 5°C. Le rendement est de 41%.

Puis, le composé **4** est obtenu en traitant le composé **3** par du pentachlorure de phosphore (2 éq.) dans l'oxychlorure de phosphore, à 110°C et pendant 18 heures. Le rendement est de 88%.

Le composé **4** ainsi obtenu réagit dans les conditions de couplage de Stille avec la 2-méthyl-6-(tributylstannyl)pyridine (4 éq.). Cette réaction est catalysée par un complexe palladié (palladium de tétrakis-(triphénylphosphine - Pd(PPh₃)₄ - 0,1 éq.) pour conduire, après 24 heures de réaction à reflux dans le toluène, à 110°C, au composé **5** avec un rendement de 85%.

Enfin, l'oxydation du composé **5** par un excès de dioxyde de sélénium (8 éq.) dans la pyridine à reflux pendant 5 jours permet d'obtenir le composé **6** avec un rendement de 98 %.

### 1.2. Synthèse du todi(ampyr)phen

Le **todi(ampyr)phen** est obtenu par réaction du composé **6** avec la di-n-octylamine.

Pour ce faire, une solution de 395 mg (0,9 mmol, 1,0 éq.) de composé **6** dans 8 mL de chlorure de thionyle (SOCl₂) est mise à chauffer à 90°C pendant 2 heures. Après évaporation du chlorure de thionyle, le résidu est repris dans 10 mL de dichlorométhane (CH₂Cl₂) et refroidi à 0°C. Sont alors ajoutés, goutte à goutte, 569 mg (5,6 mmol, 6,0 éq.) de triéthylamine (Et₃N) et 677 mg (2,8 mmol, 3,0 éq.) de di-*n*-octylamine et le mélange est agité pendant 4 heures, à température ambiante et sous atmosphère d'argon. Après évaporation à sec, le produit brut est repris dans du dichlorométhane et lavé 3 fois par contact avec de l'eau distillée. La phase organique est séchée sur sulfate de sodium (Na₂SO₄) et le solvant est évaporé. Le résidu obtenu est ensuite purifié par chromatographie sur gel de silice (élution par un mélange éther de pétrole/acétate d'éthyle 40:60).

Après évaporation des solvants, on obtient 475 mg de **todi(ampyr)phen** sous la forme d'une huile jaune. Le rendement est de 58%.
**Formule brute :** C₅₆H₈₀N₆O₂
**Masse molaire :** 868 g/mol
**RMN ¹H (400 MHz, CDCl₃) δ ppm :** 9,12 (d, *³J* = 7,8, 2H, H₆), 8,84 (d, *³J* = 8,4, 2H, H₃), 8,38 (d, *³J* = 8,4, 2H, H₂), 8,08 (t, *³J* = 7.8, 1H, H₅), 7,87 (s, 2H, H₁), 7,69 (d, *³J* = 7,8, 2H, H₄), 3,56 (q, *³J* = 7,8, 4H, 2CH₂), 3,38 (q, *³J* = 7,8, 4H, 2CH₂), 1,73 (m, 8H, 4CH₂), 1,37 (m, 20H, 10CH₂), 1,27 (m, 20H, 10CH₂), 1,09 (t, *³J* = 6,9, 6H, 2CH₃), 0,74 (t, *³J* = 6,9, 6H, 2CH₃)
**RMN ¹³C (400 MHz, CDCl₃) δ ppm :** 168,8 (2CO), 155,6 (2Cq), 154,7 (2Cq), 154,6 (2Cq), 145,6 (2Cq), 137,9 (2CH₅), 137,0 (2CH₂), 129,2 (2Cq), 126,8 (2CH₁), 123,7 (2CH₄), 122,4 (2CH₆), 120,8 (2CH₃), 49,1 (2CH₂), 46,0 (2CH₂), 31,8 (2CH₂), 31,6 (2CH₂), 29,4 (2CH₂), 29,3 (2CH₂), 29,2 (4CH₂), 29,1 (2CH₂), 27,6 (2CH₂), 27,1 (2CH₂), 26,7 (2CH₂), 22,6 (2CH₂), 22,5 (2CH₂), 14,1 (2CH₃), 13, 9 (2CH₃)
**Spectrométrie de masse (EI) , m/z (I %) :** 868, 7 (M⁺, 34%), 601,4 (M⁺-CONOct₂, 17%), 334,1 (M⁺-2CONOct₂, 100%)

### 1.3. Synthèse du dihexphdi(ampyr)phen

Le **dihexphdi(ampyr)phen** est obtenu en suivant le même protocole que celui décrit au point 1.2 ci-avant mais en faisant réagir le composé **6** avec la *N-*hexylaniline (3 éq.) en lieu et place de la di-*n-*octylamine.

Le composé **dihexphdi(ampyr)phen** est obtenu sous la forme d'une poudre beige avec un rendement de 58%.
**Formule brute :** C₄₈H₄₈N₆O₂
**Masse molaire :** 740 g/mol
**Point de fusion :** 168°C
**RMN ¹H (400 MHz, CDCl₃) δ ppm** : 8,85 (d, *³J* = 7,2, 2H, H₆), 8,20 (m, 4H, H₂, H₃) , 7,91 (m, 2H, H₅), 7,79 (s, 2H, H₁) , 7,75 (d, *³J* = 7,2, 2H, H₄), 7,15 (m, 8H, H₇, H₈), 7,01 (m, 2H, H₉), 4,01 (m, 4H, 2CH₂), 1,70 (m, 4H, 2CH₂), 1,31 (m, 12H, 6CH₂), 0,88 (m, 6H, 2CH₃)
**RMN ¹³C (400 MHz, CDCl₃) δ ppm :** 168,0 (2CO), 155,1 (2Cq), 154,0 (2Cq), 153,2 (2Cq), 145,1 (2Cq), 143,6 (2Cq), 137,4 (2CH₅), 136,7 (2CH₂ ou 2CH₃), 129,0 (2Cq), 128,7 (2CH₇ ou 2CH₈), 127,6 (2CH₇ ou 2CH₈), 126,6 (2CH₁), 126,4 (2CH₉), 124,6 (2CH₄), 122,2 (2CH₆), 120,8 (2CH₂ ou 2CH₃) , 50,5 (2CH₂), 31,5 (2CH₂), 27,5 (2CH₂), 26, 6 (2CH₂), 22,5 (2CH₂), 14,0 (2CH₃)
**Spectrométrie de masse (EI), m/z (I %) :** 740, 2 (M⁺, 14%), 334,1 (M⁺-2CONHexPh, 100%)

### 1.4. Synthèse du dietdi(ampyr)phen

Le **dietdi(ampyr)phen** est obtenu en suivant le même protocole que celui décrit au point 1.2 ci-avant mais en faisant réagir le composé **6** avec la diéthylamine (3 éq.) en lieu et place de la di-*n-*octylamine.

Le composé **dietdi(ampyr)phen** est obtenu sous la forme d'une poudre blanche avec un rendement de 52%.
**Formule brute :** C₃₂H₃₂N₆O₂
**Masse molaire :** 532 g/mol
**Point de fusion :** 201°C
**RMN ¹H (400 MHz, CDCl₃) δ ppm :** δ 9.11 (dd, *³J* = 7.8, *⁴J* = 0.6, 2H, H₆), 8.88 (d, *³J* = 8.4, 2H, H₃), 8.42 (d, *³J* = 8.4, 2H, H₂), 8.10 (t, *³J* = 7.8, 2H, H₅), 7.89 (s, 2H, H₁), 7.72 (dd, *³J* = 7.8, *⁴J* = 0.6, 2H, H₄), 3.65 (q, *³J* = 6. 9, 4H, 2CH₂), 3. 47 (q, *³J* = 6. 9, 4H, 2CH₂), 1.34 (t, *³J* = 6.9, 6H, 2CH₃), 1.27 (t, *³J* = 6.9, 6H, 2CH₃)
**RMN ¹³C (400 MHz, CDCl₃) δ ppm :** δ 168.2 (2CO), 155.1 (2Cq), 154.2 (2Cq), 154.1 (2Cq), 145.0 (2Cq), 137.6 (2C₅), 136.9 (2C₂), 128.8 (2Cq), 126.5 (2C₁), 123.3 (2C₄), 122.2 (2C₆), 120.5 (2C₃), 42.9 (2CH₂), 39.9 (2CH₂), 14.1 (2CH₃), 12.5 (2CH₃)
**Spectrométrie de masse (EI), m/z (I %)** : 532 (M⁺, 42%), 334 (M⁺-CONEt₂, 100%)

### 1.5. Synthèse du dietphdi(ampyr)phen

Le **dietphdi(ampyr)phen** est obtenu en suivant le même protocole que celui décrit au point 1.2 ci-avant mais en faisant réagir le composé **6** avec la N-éthylaniline (3 éq.) en lieu et place de la di-n-octylamine.

Le composé **dietphdi(ampyr)phen** est obtenu sous la forme d'une poudre blanche avec un rendement de 31%.
**Formule brute :** C₄₀H₃₂N₆O₂
**Masse molaire :** 628 g/mol
**Point de fusion :** > 230°C
**RMN ¹H (400 MHz, CDCl₃) δ ppm :** δ 8.90 (d, *³J* = 7.8, 2H, H₆), 8.35 (m, 4H, H₂, H₃) , 7.95 (t, *³J* = 7.8, 2H, H₅), 7.86 (s, 2H, H₁), 7.77 (d, *³J* = 7.8, 2H, H₄), 7.16 (m, 8H, H₇, H₈) , 7.03 (m, 2H, H₉), 4.11 (q, *³J* = 7.2, 4H, CH₂), 1.31 (t, *³J* = 7.2, 6H, CH₃)
**RMN ¹³C (400 MHz_{,} CDCl₃) δ ppm :** δ 167.8 (2CO), 155.2 (2Cq), 154.2 (2Cq), 153.1 (2Cq), 145.2 (2Cq), 143.4 (2Cq), 137.4 (2C₅), 136.6 (2C₂ or 2 C₃), 129.0 (2Cq) , 128.8 (4C₇ or 4C₈), 127.7 (4C₇ or 4C₈) , 126.6 (2C₁), 126.5 (2C₉), 124.6 (2C₄), 122.3 (2C₆), 120.8 (2C₂ or 2C₃), 45.5 (2CH₂), 12.8 (2CH₃)
**Spectrométrie de masse (EI), m/z (I %) :** 628 (M⁺, 21%), 418 (M⁺-CONEtPh, 14%), 334 (M⁺-2CONEtPh, 100%)

### EXEMPLE 2 : PROPRIETES EXTRACTANTES DES COMPOSES SELON L'INVENTION

Des extractions liquide-liquide sont réalisées en utilisant :
- comme phases organiques : deux solutions comprenant l'une du **todi(ampyr)phen** et l'autre du **dihexphdi(ampyr)phen** à 0,01 mol/L dans du *n*-octanol ; et
- comme phases aqueuses : trois solutions aqueuses dénommées ci-après S1, S2 et S3 et comprenant respectivement :
   S1 : un mélange de ²³⁹⁻²⁴⁰Pu (IV), ²⁴¹Am(III), ²⁴⁴Cm(III) et ¹⁵²Eu(III), tous à l'état de traces, c'est-à-dire à hauteur de 10⁻⁵ à 10⁻⁶ mol/L, dans de l'acide nitrique à 3 mol/L ;
   S2 : du ²³⁷Np(V) à 5.10⁻⁴ mol/L dans de l'acide nitrique à 3 mol/L ;
   S3 : du ²³⁸U(VI) à 5.10⁻³ mol/L dans de l'acide nitrique à 3 mol/L.

Chaque phase organique est mise en contact avec chacune des phases aqueuses S1, S2 et S3, à raison d'un volume de phase organique pour un volume de phase aqueuse, et les phases ainsi mises en contact sont laissées sous agitation mécanique pendant une heure à une température constante de 25°C.

Après quoi, les phases organique et aqueuse sont séparées l'une de l'autre et les activités des différents radioéléments sont déterminées dans chacune de ces phases par spectrométrie α, spectrométrie γ ou par fluorescence X selon le cas.

Le tableau I ci-après présente, pour chaque type de phase organique et pour chaque extraction, les coefficients de distribution du plutonium(IV), de l'américium(III), du curium(III), de l'europium(III), du neptunium(V) et de l'uranium(VI) obtenus à partir des activités ainsi déterminées, tandis que le tableau II ci-après présente, pour chaque type de phase organique, les facteurs de séparation FS_{Pu/Eu}, FS_{Am/Eu}, FS_{Cm/Ce} et FS_{Am/Cm} obtenus à partir de ces coefficients de distribution.

On rappelle que, dans le domaine des extractions liquide-liquide, le coefficient de distribution, noté D_{M}, d'un élément M correspond au rapport, à l'équilibre, des concentrations (représentées ici par les activités) de cet élément dans les phases organique et aqueuse ayant été mises en contact, et que le facteur de séparation entre deux éléments métalliques M1 et M2, noté FS_{M1/M2}, correspond à D_{M1}/D_{M2}, c'est-à-dire au rapport des coefficients de distribution des éléments métalliques M1 et M2 obtenus au cours d'une même extraction.

**Tableau I**

| **D_{M}** | **Phase organique** | |
|---|---|---|
| | **todi(ampyr)phen** | **dihexphdiampyrphen** |
| **D_{Pu(IV)}** | 2,17 | 14,80 |
| **D_{Am(III)}** | 0,17 | 1,75 |
| **D_{Cm(III)}** | 0,07 | 0,74 |
| **D_{Eu(III)}** | 0,0063 | 0,091 |
| **D_{Np(V)}** | 11,01 | 16,60 |
| **D_{U(VI)}** | 1,92 | > 2 |

**Tableau II**

| **FS_{M1/M2}** | **Phase organique** | |
|---|---|---|
| | **todi(ampyr)phen** | **dihexphdiampyrphen** |
| **FS_{Pu/Eu}** | 349 | 166 |
| **FS_{Am/Eu}** | 25 | 19 |
| **FS_{Cm/Eu}** | 10 | 8 |
| **FS_{Am/Cm}** | 2,3 | 2,3 |

Ces tableaux montrent que les composés selon l'invention extraient les actinides(III) (IV), (V) et (VI) d'une solution aqueuse d'acidité élevée (3 mol/L) beaucoup plus efficacement que ne le font les terpyridines proposées dans la référence [4] précitée puisque :
- les coefficients de distribution du plutonium(IV) sont tous égaux ou supérieurs à 2 ;
- les coefficients de distribution du neptunium(V) sont tous égaux ou supérieurs à 11 ;
- les coefficients de distribution de l'uranium(VI) sont supérieurs à 1,9 ; tandis que
- les coefficients de distribution de l'américium et du curium sont tous égaux ou supérieurs à 0,07.

Ces tableaux montrent également que les composés selon l'invention extraient les actinides(III) et (IV) d'une telle solution beaucoup plus efficacement qu'ils n'extraient les lanthanides puisque :
- les facteurs de séparation entre le plutonium(IV) et l'europium sont tous supérieurs à 160 ; tandis que
- les facteurs de séparation entre les actinides(III) et l'europium sont tous égaux ou supérieurs à 8.

Ces tableaux montrent encore que les composés selon l'invention présentent une affinité plus marquée pour l'américium que pour le curium puisque les coefficients de distribution de l'américium sont tous 2,3 fois supérieurs à ceux obtenus pour le curium.

### REFERENCES CITEES

[1] Demande internationale PCT WO 2007/118904
[2] Demande internationale PCT WO 2008/049807
[3] Aneheim et al., Solv. Ext. Ion Exch. (2010), 28, 437-458
[4] Demande internationale PCT WO 2011/009814
[5] Yamada et al., Bulletin of Chemical Society of Japan (1990), 63, 2710-2712

## Revendications

1. Composé, qui répond à la formule générale (I) ci-après : 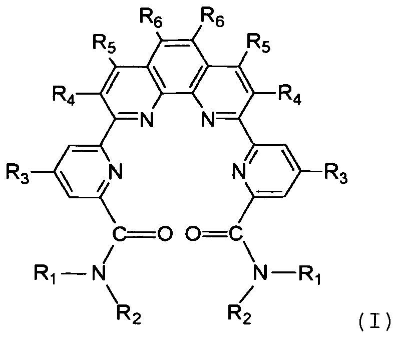 dans laquelle :
R₁ et R₂, identiques ou différents, représentent un atome d'hydrogène, un groupe hydrocarboné linéaire ou ramifié, saturé ou insaturé, en C₁ à C₁₂, ou un groupe aryle ou aryl-alkyle en C₁ à C₆ monocyclique ;
R₃, R₄, R₅ et R₆, identiques ou différents, représentent :
* un atome d'hydrogène ; ou
* un groupe hydrocarboné linéaire ou ramifié, saturé ou insaturé, en C₁ à C₁₂ ; ou
* un groupe aryle ou aryl-alkyle en C₁ à C₆ monocyclique ; ou
* un groupe -NR₇R₈ ou -NR₇COR₈ dans lequel R₇ représente un atome d'hydrogène, un groupe hydrocarboné linéaire ou ramifié, saturé ou insaturé, en C₁ à C₁₂, ou un groupe aryle ou aryl-alkyle en C₁ à C₆ monocyclique, tandis que R₈ représente un groupe hydrocarboné linéaire ou ramifié, saturé ou insaturé, en C₁ à C₁₂, ou un groupe aryle ou aryl-alkyle en C₁ à C₆ monocyclique ; ou encore
* un groupe -OR₉ ou -SR₉ dans lequel R₉ représente un groupe hydrocarboné linéaire ou ramifié, saturé ou insaturé, en C₁ à C₁₂, ou un groupe aryle ou aryl-alkyle en C₁ à C₆ monocyclique.

2. Composé selon la revendication 1, qui répond à la formule générale (I) dans laquelle :
R₁ et R₂, identiques ou différents, représentent un atome d'hydrogène, un groupe alkyle linéaire ou ramifié en C₁ à C₁₂ ou un groupe aryle monocyclique ;
R₃, R₄, R₅ et R₆, identiques ou différents, représentent :
* un atome d'hydrogène ; ou
* un groupe alkyle linéaire ou ramifié en C₁ à C₁₂ ; ou
* un groupe aryle monocyclique ; ou
* un groupe -NR₇R₈ ou -NR₇COR₈ dans lequel R₇ représente un atome d'hydrogène, un groupe alkyle linéaire ou ramifié en C₁ à C₁₂, ou un groupe aryle monocyclique, tandis que R₈ représente un groupe alkyle linéaire ou ramifié en C₁ à C₁₂ ou un groupe aryle monocyclique ; ou
* un groupe -OR₉ ou -SR₉ dans lequel R₉ représente un groupe alkyle linéaire ou ramifié en C₁ à C₁₂, ou un groupe aryle monocyclique.

3. Composé selon l'une quelconque des revendications précédentes, qui répond à la formule générale (I) dans laquelle R₃, R₄, R₅ et R₆ représentent un atome d'hydrogène.

4. Composé selon l'une quelconque des revendications précédentes, qui répond à la formule générale (I) dans laquelle R₁ et R₂, identiques ou différents, représentent soit une chaîne alkyle en C₁ à C₁₂, et mieux encore en C₂ à C₁₂, soit un groupe phényle, soit encore un groupe *ortho-, méta-* ou *para*-tolyle.

5. Composé selon l'une quelconque des revendications précédentes, qui est choisi parmi :
- le *N,N,N',N'*-tétraéthyl-6, 6'-([1,10]-phénanthrolin-2,9-diyl)-pyridin-2-yldiamide ;
- le *N,N,N',N'*-tétrahexyl-6,6'-([1,10]-phénanthrolin-2,9-diyl)-pyridin-2-yldiamide ;
- le *N,N,N',N'*-tétraoctyl-6,6'-([1,10]-phénanthrolin-2,9-diyl)-pyridin-2-yldiamide ;
- le *N,N,N',N'*-tétradécyl-6,6'-([1,10]-phénanthrolin-2,9-diyl)-pyridin-2-yldiamide ;
- le *N,N,N',N'*-tétradodécyl-6,6'-([1,10]-phénanthrolin-2,9-diyl)-pyridin-2-yldiamide ;
- le *N,N'*-diéthyl-*N,N'*-diphényl-6,6'-([1,10]-phénanthrolin-2,9-diyl)-pyridin-2-yldiamide ;
- le *N,N'*-dihexyl-*N,N'*-diphényl-6,6'-([1,10]-phénanthrolin-2,9-diyl)-pyridin-2-yldiamide ;
- le *N,N'*-dioctyl-*N,N'*-diphényl-6,6'-([1,10]-phénanthrolin-2,9-diyl)-pyridin-2-yldiamide ;
- le *N,N'*-didécyl-*N,N'*-diphényl-6,6'-([1,10]-phénanthrolin-2,9-diyl)-pyridin-2-yldiamide ; et
- le *N,N*'-didodécyl-*N,N'*-diphényl-6,6'-([1,10]-phénanthrolin-2,9-diyl)-pyridin-2-yldiamide.

6. Procédé de synthèse d'un composé tel que défini dans l'une quelconque des revendications 1 à 5, qui comprend les étapes suivantes :
- la réaction d'un composé répondant à la formule générale (II) ci-après : 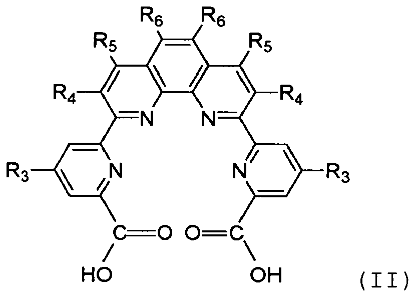 dans laquelle R₃, R₄, R₅ et R₆, identiques ou différents, ont la même signification que dans la formule générale (I), avec un réactif d'halogénation pour obtenir un composé répondant à la formule générale (III) ci-après : 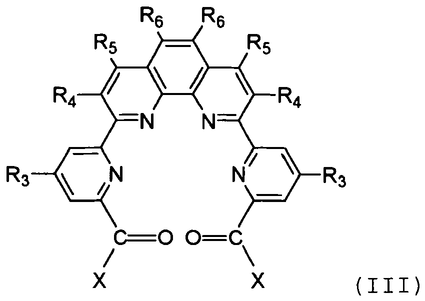 dans laquelle R₃, R₄, R₅ et R₆, identiques ou différents, ont la même signification que dans la formule générale (II) tandis que X représente un atome d'halogène ; et
- la réaction du composé répondant à la formule (III) avec une amine de formule HNR₁R₂ dans laquelle R₁ et R₂ ont la même signification que dans la formule générale (I), cette réaction étant réalisée en présence d'une base et dans un solvant polaire aprotique.

7. Procédé selon la revendication 6, dans lequel le réactif d'halogénation est choisi parmi le chlorure de thionyle, l'oxychlorure de phosphore et le trichlorure de phosphore, tandis que la base est choisie parmi la triéthylamine, la diisopropyl-éthylamine et la pyridine.

8. Utilisation d'un composé selon l'une quelconque des revendications 1 à 5, en tant que ligand d'un ou plusieurs actinides.

9. Utilisation selon la revendication 8, dans laquelle le composé est utilisé pour extraire un ou des actinides d'une solution aqueuse acide.

10. Utilisation selon la revendication 9, dans laquelle le composé est utilisé pour séparer l'ensemble des actinides présents dans une solution aqueuse acide des lanthanides qui sont également présents dans cette solution.

11. Utilisation selon la revendication 10, dans laquelle la solution aqueuse acide est une solution résultant de la dissolution d'un combustible nucléaire usé dans de l'acide nitrique.

12. Utilisation selon la revendication 11, dans laquelle la solution aqueuse acide est une solution résultant de la dissolution d'un combustible nucléaire usé dans de l'acide nitrique mais ayant été préalablement débarrassée de l'uranium qu'elle contenait initialement.

13. Utilisation selon l'une quelconque des revendications 9 à 12, qui comprend les étapes suivantes :
- l'extraction de l'ensemble des actinides de la solution aqueuse acide par mise en contact de cette solution aqueuse avec une phase organique comprenant le composé puis séparation de ladite solution aqueuse et de ladite phase organique ; et
- la désextraction de l'ensemble des actinides présents dans la phase organique obtenue à l'issue de l'étape d'extraction par mise en contact de cette phase organique avec une phase aqueuse acide, de préférence de pH allant de 2 à 3, puis séparation desdites phases organique et aqueuse.

14. Utilisation selon la revendication 9, dans laquelle le composé est utilisé pour séparer l'américium présent dans une solution aqueuse acide du curium qui est également présent dans cette solution.

15. Utilisation selon la revendication 14, qui comprend les étapes suivantes :
- l'extraction de l'américium de la solution aqueuse acide par mise en contact de cette solution aqueuse avec une phase organique comprenant le composé puis séparation de ladite solution aqueuse et de ladite phase organique ; et
- la désextraction de l'américium présent dans la phase organique obtenue à l'issue de l'étape d'extraction par mise en contact de cette phase organique avec une phase aqueuse acide, de préférence de pH allant de 2 à 3, puis séparation desdites phases organiques et aqueuse.

16. Utilisation selon l'une quelconque des revendications 9 à 15, dans laquelle le composé est utilisé en solution dans un solvant organique choisi parmi le n-octanol, le n-dodécane et le tétrapropylène hydrogéné.

## Patentansprüche

1. Verbindung, die der nachfolgenden allgemeinen Formel (I) entspricht: 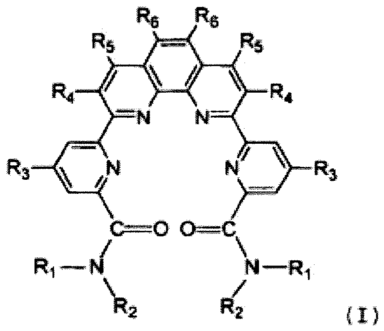 wobei:
R₁ und R₂, die identisch oder unterschiedlich sind, ein Wasserstoffatom, eine lineare oder verzweigte, gesättigte oder ungesättigte C₁ bis C₁₂ Kohlenwasserstoffgruppe, oder eine monozyklische C₁ bis C₆ Aryl- oder Aryl-Alkylgruppe darstellen;
R₃, R₄, R₅ und R₆, die identisch oder unterschiedlich sind, folgendes darstellen:
* ein Wasserstoffatom; oder
* eine lineare oder verzweigte, gesättigte oder ungesättigte C₁ bis C₁₂ Kohlenwasserstoffgruppe; oder
* eine monozyklische C₁ bis C₆ Aryl- oder Aryl-Alkyl-Gruppe; oder
* eine Gruppe -NR₇R₈ oder -NR₇COR₈, wobei R₇ ein Wasserstoffatom, eine lineare oder verzweigte, gesättigte oder ungesättigte C₁ bis C₁₂ Kohlenwasserstoffgruppe oder eine monozyklische C₁ bis C₆ Aryl- oder Aryl-Alkyl-Gruppe darstellt, wohingegen R₈ eine lineare oder verzweigte, gesättigte oder ungesättigte C₁ bis C₁₂ Kohlenwasserstoffgruppe oder eine monozyklische C₁ bis C₆ Aryl- oder Aryl-Alkyl-Gruppe darstellt;
oder aber:
* eine Gruppe -OR₉ oder -SR₉, wobei R₉ eine lineare oder verzweigte, gesättigte oder ungesättigte C₁ bis C₁₂ Kohlenwasserstoffgruppe oder eine monozyklische C₁ bis C₆ Aryl- oder Aryl-Alkyl-Gruppe darstellt.

2. Verbindung nach Anspruch 1, die der allgemeinen Formel (I) entspricht, wobei:
R₁ und R₂, die identisch oder unterschiedlich sind, ein Wasserstoffatom, eine lineare oder verzweigte C₁ bis C₁₂ Alkylgruppe oder eine monozyklische Arylgruppe darstellen;
R₃, R₄, R₅ und R₆, die identisch oder unterschiedlich sind, folgendes darstellen:
* ein Wasserstoffatom; oder
* eine lineare oder verzweigte C₁ bis C₁₂ Alkylgruppe; oder
* eine monozyklische Arylgruppe; oder
* eine Gruppe -NR₇R₈ oder -NR₇COR₈, wobei R₇ ein Wasserstoffatom, eine lineare oder verzweigte C₁ bis C₁₂ Alkylgruppe oder eine monozyklische Arylgruppe darstellt, wohingegen R₈ eine lineare oder verzweigte C₁ bis C₁₂ Alkylgruppe oder eine monozyklische Arylgruppe darstellt; oder
* eine Gruppe -OR₉ oder -SR₉, wobei R₈ eine lineare oder verzweigte C₁ bis C₁₂ Alkylgruppe oder eine monozyklische Arylgruppe darstellt.

3. Verbindung nach einem der vorhergehenden Ansprüche, die der allgemeinen Formel (I) entspricht, wobei R₃, R₄, R₅ und R₆ ein Wasserstoffatom darstellen.

4. Verbindung nach einem der vorhergehenden Ansprüche, die der allgemeinen Formel (I) entspricht, wobei R₁ und R₂, die identisch oder unterschiedlich sind, entweder eine C₁ bis C₁₂ und bevorzugt eine C₂ bis C₁₂ Alkylkette oder eine Phenylgruppe oder auch eine Ortho-, Meta- oder Para-Tolylgruppe darstellen.

5. Verbindung nach einem der vorhergehenden Ansprüche, die ausgewählt ist aus:
- N, N, N', N'-tetraethyl-6, 6'-([1,10]-phenanthrolin-2,9-diyl)-pyridin-2-yldiamid;
- N, N, N', N'-tetrahexyl-6, 6'-([1,10]-phenanthrolin-2, 9-diyl)-pyridin-2-yldiamid;
- N, N, N', N'-tetraoctyl-6, 6'-([1,10]-phenanthrolin-2,9-diyl)-pyridin-2-yldiamid;
- N, N, N', N'-tetradecyl-6, 6'-([1,10]-phenanthrolin-2, 9-diyl)-pyridin-2-yldiamid;
- N, N, N', N'-tetradodecyl-6, 6'-([1,10]-phenanthrolin-2,9-diyl)-pyridin-2-yldiamid;
- N, N'-diethyl-N, N'-diphenyl-6, 6'-([1,10]-phenanthrolin-2,9-diyl)-pyridin-2-yldiamid;
- N, N'-dihexyl-N, N'-diphenyl-6, 6'-([1,10]-phenanthrolin-2, 9-diyl)-pyridin-2-yldiamid;
- N, N'-dioctyl-N, N'-diphenyl-6, 6'-([1,10]-phenanthrolin-2, 9-diyl)-pyridin-2-yldiamid;
- N, N'-didecyl-N, N'-diphenyl-6, 6'-([1,10]-phenanthrolin-2,9-diyl)-pyridin-2-yldiamid;
und
- N, N'-didodecyl-N, N'-diphenyl-6, 6'-([1,10]-phenanthrolin-2, 9-diyl)-pyridin-2-yldiamid.

6. Verfahren zur Synthese einer Verbindung, wie sie in einem der Ansprüche 1 bis 5 definiert ist, umfassend die folgenden Schritte:
- Die Reaktion einer Verbindung, welche der nachfolgenden allgemeinen Formel (II) entspricht: 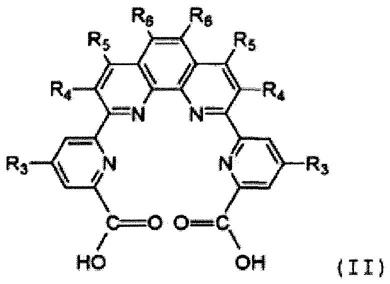 wobei R₃, R₄, R₅ und R₆, die identisch oder unterschiedlich sind, die gleiche Bedeutung wie in der allgemeinen Formel (I) haben, mit einem Halogenierungsreagenz, um eine Verbindung zu erhalten, die der nachfolgenden allgemeinen Formel (III) entspricht: 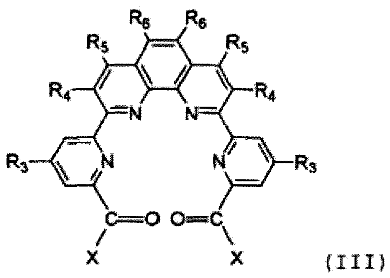 wobei R₃, R₄, R₅ und R₆, die identisch oder unterschiedlich sind, die gleiche Bedeutung haben wie in der allgemeinen Formel (II), wohingegen X ein Halogenatom darstellt; und
- Die Reaktion der Verbindung, die der Formel (III) entspricht, mit einem Amin der Formel HNR₁R₂, wobei R₁ und R₂ die gleiche Bedeutung wie in der allgemeinen Formel (I) haben, wobei diese Reaktion in Anwesenheit einer Base und in einem aprotischen polaren Lösungsmittel durchgeführt wird.

7. Verfahren nach Anspruch 6, wobei das Halogenierungsreagenz ausgewählt ist aus Thionylchlorid, Phosphoroxychlorid und Phosphortrichlorid, wohingegen die Base ausgewählt ist aus Triethylamin, Diisopropyl-Ethylamin und Pyridin.

8. Verwendung einer Verbindung nach einem der Ansprüche 1 bis 5 als Ligand von einem oder mehreren Aktiniden.

9. Verwendung nach Anspruch 8, wobei die Verbindung verwendet wird, um ein oder mehrere Aktiniden aus einer sauren wässrigen Lösung zu extrahieren.

10. Verwendung nach Anspruch 9, wobei die Verbindung verwendet wird, um die Gesamtheit der Actiniden, die in einer sauren wässrigen Lösung vorhanden sind, von den Lanthaniden zu trennen, die ebenfalls in dieser Lösung vorhanden sind.

11. Verwendung nach Anspruch 10, wobei die saure wässrige Lösung eine Lösung ist, die aus der Auflösung eines gebrauchten Kernbrennstoffs in Salpetersäure resultiert.

12. Verwendung nach Anspruch 11, wobei die saure wässrige Lösung eine Lösung ist, die aus der Auflösung eines gebrauchten Kernbrennstoffs in Salpetersäure resultiert, aus der jedoch vorher das Uran entfernt wurde, welches anfänglich enthalten war.

13. Verwendung nach einem der Ansprüche 9 bis 12, die die folgenden Schritte umfasst:
- die Extraktion der Gesamtheit der Aktiniden aus der sauren wässrigen Lösung durch in Kontakt bringen dieser wässrigen Lösung mit einer organischen Phase, welche die Verbindung enthält, und anschließende Trennung der wässrigen Lösung und der organischen Phase; und
- die Desextraktion der Gesamtheit der Aktiniden, die in der organischen Phase vorhanden sind, welche am Ende des Schritts der Extraktion erhalten wird, durch in Kontakt bringen dieser organischen Phase mit einer sauren wässrigen Phase, vorzugsweise mit einem ph-Wert zwischen 2 und 3, und anschließende Trennung der organischen und der wässrigen Phase.

14. Verwendung nach Anspruch 9, wobei die Verbindung verwendet wird, um das Americium, das in einer sauren wässrigen Lösung vorhanden ist, von dem Curium zu trennen, welches ebenfalls in dieser Lösung vorhanden ist.

15. Verwendung nach Anspruch 14, die die folgenden Schritte umfasst:
- Die Extraktion des Americiums aus der sauren wässrigen Lösung durch in Kontakt bringen dieser wässrigen Lösung mit einer organischen Phase, die die Verbindung enthält, und anschließende Trennung der wässrigen Lösung und der organischen Phase; und
- die Desextraktion des Americiums, das in der organischen Phase vorhanden ist, welche am Ende des Schritts der Extraktion erhalten wird, durch in Kontakt bringen dieser organischen Phase mit einer sauren wässrigen Phase, vorzugsweise mit einem ph-Wert zwischen 2 und 3, und anschließende Trennung der organischen und der wässrigen Phase.

16. Verwendung nach einem der Ansprüche 9 bis 15, wobei die Verbindung in Lösung in einem organischen Lösungsmittel verwendet wird, welches ausgewählt ist aus n-Octanol, n-Dodecan und wasserstoffhaltigem Tetrapropylen.

## Claims

1. Compound which meets the general formula (I) hereinafter: 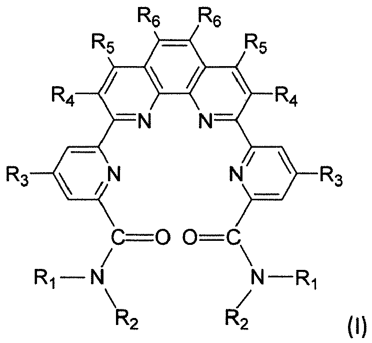 in which:
R₁ and R₂, either identical or different, represent a hydrogen atom, a C₁ to C₁₂ saturated or unsaturated, straight-chain or branched hydrocarbon group, or a monocyclic aryl or aryl-(C₁ to C₆)alkyl group;
R₃, R₄, R₅ and R₆, either identical or different, represent:
* a hydrogen atom; or
* a C₁ to C₁₂ saturated or unsaturated straight-chain or branched hydrocarbon group; or
* a monocyclic aryl or aryl-(C₁ to C₆)alkyl group; or
* a -NR₇R₈ or -NR₇COR₈ group in which R₇ represents a hydrogen atom, a C₁ to C₁₂ saturated or unsaturated, straight-chain or branched hydrocarbon group, or a monocyclic aryl or aryl-(C₁ to C₆)alkyl group, whilst R₈ represents a C₁ to C₁₂ saturated or unsaturated, straight-chain or branched hydrocarbon group, or a monocyclic aryl or aryl-(C₁ to C₆)alkyl group; or still
* an -OR₉ or -SR₉ group in which R₉ represents a C₁ to C₁₂ saturated or unsaturated, straight-chain or branched hydrocarbon group, or a monocyclic aryl or aryl-(C₁ to C₆)alkyl group.

2. Compound according to claim 1, which meets the general formula (I) in which:
R₁ and R₂, either identical or different, represent a hydrogen atom, a C₁ to C₁₂ straight-chain or branched alkyl group or a monocyclic aryl group;
R₃, R₄, R₅ and R₆, either identical or different, represent:
* a hydrogen atom; or
* a C₁ to C₁₂ straight-chain or branched alkyl group; or
* a monocyclic aryl group; or
* a -NR₇R₈ or -NR₇COR₈ group in which R₇ represents a hydrogen atom, a C₁ to C₁₂ straight-chain or branched alkyl group, or a monocyclic aryl group, whilst R₈ represents a C₁ to C₁₂ straight-chain or branched alky group or a monocyclic aryl group; or still
* an -OR₉ or -SR₉ group in which R₉ represents a C₁ to C₁₂ straight-chain or branched alkyl group, or a monocyclic aryl group.

3. Compound according to any of the preceding claims, which meets the general formula (I) in which R₃, R₄, R₅ and R₆ represent a hydrogen atom.

4. Compound according to any of the preceding claims, which meets the general formula (I) in which R₁ and R₂, either identical or different, represent either a C₁ to C₁₂, and better still C₂ to C₁₂, alkyl chain, or a phenyl group or still an *ortho-, meta-* or *para-*tolyl group.

5. Compound according to any of the preceding claims, which is chosen from among:
- *N,N,N,N'*-tetraethyl-6,6'-([1,10]-phenanthrolin-2,9-diyl)-pyridin-2-yldiamide;
- *N,N,N',N'*-tetrahexyl-6,6'-([1,10]-phenanthrolin-2,9-diyl)-pyridin-2-yldiamide;
- *N,N,N',N'*-tetraoctyl-6,6'-([1,10]-phenanthrolin-2,9-diyl)-pyridin-2-yldiamide;
- *N,N,N',N'*-tetradecyl-6,6'-([1,10]-phenanthrolin-2,9-diyl)-pyridin-2-yldiamide;
- *N,N,N',N'*-tetradodecyl-6,6'-([1,10]-phenanthrolin-2,9-diyl)-pyridin-2-yldiamide;
- *N,N'*-diethyl-*N,N'*-diphenyl-6,6'-([1,10]-phenanthrolin-2,9-diyl)-pyridin-2-yldiamide;
- *N,N'*-dihexyl-*N,N'*-diphenyl-6,6'-([1,10]-phenanthrolin-2,9-diyl)-pyridin-2-yldiamide;
- *N,N'*-dioctyl-*N,N'*-diphenyl-6,6'-([1,10]-phenanthrolin-2,9-diyl)-pyridin-2-yldiamide;
- *N,N'*-didecyl-*N,N'*-diphenyl-6,6'-([1,10]-phenanthrolin-2,9-diyl)-pyridin-2-yldiamide; and
- *N,N'*-didodecyl-*N,N'*-diphenyl-6,6'-([1,10]-phenanthrolin-2,9-diyl)-pyridin-2-yldiamide.

6. Method for synthesizing a compound as defined in any of claims 1 to 5, which comprises the following steps:
- reacting a compound meeting the general formula (II) hereinafter: 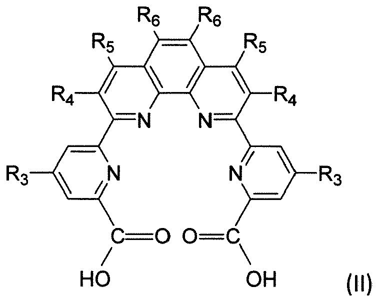 in which R₃, R₄, R₅ and R₆, either identical or different, have the same meaning as in the general formula (I), with a halogenation reagent to obtain a compound meeting the general formula (III) hereinafter: 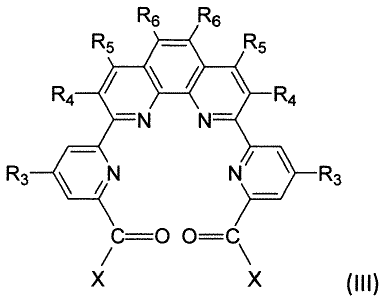 in which R₃, R₄, R₅ and R₆, either identical or different, have the same meaning as in the general formula (II) whilst X represents a halogen atom; and
- reacting the compound meeting the formula (III) with an amine of formula HNR₁R₂ in which R₁ and R₂ have the same meaning as in the general formula (I), this reaction taking place in the presence of a base and in an aprotic polar solvent.

7. Method according to claim 6, wherein the halogenation reagent is chosen from among thionyl chloride, phosphorus oxichloride and phosphorus trichloride, whilst the base is chosen from among triethylamine, diisopropylethylamine and pyridine.

8. Use of a compound according to any of claims 1 to 5, as a ligand for one or more actinides.

9. Use according to claim 8, wherein the compound is used to extract one or more actinides from an acid aqueous solution.

10. Use according to claim 9, wherein the compound is used to separate all the actinides present in an acid aqueous solution from the lanthanides also contained in this solution.

11. Use according to claim 10, wherein the acid aqueous solution is a solution resulting from the dissolution of a spent nuclear fuel in nitric acid.

12. Use according to claim 11, wherein the acid aqueous solution is a solution resulting from the dissolution of a spent nuclear fuel in nitric acid but previously rid of the uranium it initially contained.

13. Use according to any of claims 9 to 12, which comprises the following steps:
- extracting all the actinides from the acid aqueous solution by contacting this aqueous solution with an organic phase comprising the compound, then separating said aqueous solution and said organic phase; and
- stripping all the actinides present in the organic phase obtained at the end of the extraction step, by contacting this organic phase with an acid aqueous phase preferably having a pH ranging from 2 to 3, then separating said organic and aqueous phases.

14. Use according to claim 9, wherein the compound is used to separate the americium present in an acid aqueous solution from the curium which is also contained in this solution.

15. Use according to claim 14, which comprises the following steps:
- extracting the americium from the acid aqueous solution by contacting this aqueous solution with an organic phase comprising the compound, then separating said aqueous solution and said organic phase; and
- stripping the americium present in the organic phase obtained at the end of the extraction step, by contacting this organic phase with an acid aqueous phase preferably having a pH ranging from 2 to 3, then separating said organic and aqueous phases.

16. Use according to any of claims 9 to 15, wherein the compound is used in solution in an organic solvent chosen from among n-octanol, n-dodecane and hydrogenated tetrapropylene.
